(19) Europäisches Patentamt
European Patent Office
Office européen des brevets

(11) **EP 3 438 986 B1**

(12) **EUROPEAN PATENT SPECIFICATION**

(45) Date of publication and mention
of the grant of the patent:
**10.07.2024  Bulletin 2024/28**

(21) Application number: **17306035.1**

(22) Date of filing: **02.08.2017**

(51) International Patent Classification (IPC):
**G16H 20/17** (2018.01)   **G16H 40/40** (2018.01)
**G16H 50/50** (2018.01)   **A61B 5/145** (2006.01)
**A61M 5/142** (2006.01)   **A61M 5/172** (2006.01)

(52) Cooperative Patent Classification (CPC):
**G16H 20/17; A61B 5/14532; G16H 40/40;
G16H 50/50;** A61M 2005/14208; A61M 2005/1726;
A61M 2230/201

(54) **CLOSED-LOOP BLOOD GLUCOSE CONTROL SYSTEMS AND METHODS**

BLUTGLUCOSEKONTROLLSYSTEME UND -VERFAHREN MIT GESCHLOSSENER SCHLEIFE

SYSTÈMES ET PROCÉDÉS DE CONTRÔLE DE GLYCÉMIE À BOUCLE FERMÉE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**06.02.2019  Bulletin 2019/06**

(73) Proprietor: **Diabeloop
38028 Grenoble Cedex 1 (FR)**

(72) Inventors:
• **Desir, Chesner
155-157 Cours Berriat
38000 Grenoble (FR)**
• **Lachal, Sylvain
155-157 Cours Berriat
38000 Grenoble (FR)**
• **Franco, Celine
155-157 Cours Berriat
38000 Grenoble (FR)**

(74) Representative: **Pellegri, Michel Pascal Romain
Cabinet Vulpelex
52 Rue Montgolfier
69006 Lyon (FR)**

(56) References cited:
**WO-A2-2007/116226      US-A1- 2016 354 543**

• **"DTU Compute-Technical Report", vol. 13, 2013,
TECHNICAL UNIVERSY OF DENMARK, ISSN:
1601-2321, article ANNE KATRINE
DUUN-HENRIKSEN ET AL: "Modelling the Effect
of Exercise on Insulin Pharmacokinetics
in "Continuous Subcutaneous Insulin
Infusion" Treated Type 1 Diabetes Patients",
XP055438255**
• **DIMITRI BOIROUX ET AL: "Model Predictive
Control Algorithms for Pen and Pump Insulin
Administration", 20 October 2015 (2015-10-20),
pages 15 - 24, XP002776941, Retrieved from the
Internet
<URL:http://orbit.dtu.dk/files/57023470/phd283_
Boiroux_D.pdf> [retrieved on 20171113]**
• **SOEBORG T ET AL: "Absorption kinetics of
insulin after subcutaneous administration",
EUROPEAN JOURNAL OF PHARMACEUTICAL
SCIENCES, ELSEVIER AMSTERDAM, NL, vol. 36,
no. 1, 31 January 2009 (2009-01-31), pages 78 -
90, XP025860044, ISSN: 0928-0987, [retrieved on
20081105], DOI: 10.1016/J.EJPS.2008.10.018**
• **ROMAN HOVORKA ET AL: "Nonlinear model
predictive control of glucose concentration in
subjects with type 1 diabetes; Controlling
glucose", PHYSIOLOGICAL MEASUREMENT,
INSTITUTE OF PHYSICS PUBLISHING, BRISTOL,
GB, vol. 25, no. 4, 1 August 2004 (2004-08-01),
pages 905 - 920, XP020074167, ISSN: 0967-3334,
DOI: 10.1088/0967-3334/25/4/010**

**(Cont. next page)**

- **JALLON P ET AL: "Personalization of a compartmental physiological model for an artificial pancreas through integration of patient's state estimation", 2017 39TH ANNUAL INTERNATIONAL CONFERENCE OF THE IEEE ENGINEERING IN MEDICINE AND BIOLOGY SOCIETY (EMBC), IEEE, 11 July 2017 (2017-07-11), pages 1453 - 1456, XP033152274, DOI: 10.1109/EMBC.2017.8037108**
- **DATABASE MEDLINE [online] US NATIONAL LIBRARY OF MEDICINE (NLM), BETHESDA, MD, US; 2018, GRADEL A K J ET AL: "Factors Affecting the Absorption of Subcutaneously Administered Insulin: Effect on Variability.", Database accession no. NLM30116732**
- **GRADEL A K J ET AL: "Factors Affecting the Absorption of Subcutaneously Administered Insulin: Effect on Variability.", JOURNAL OF DIABETES RESEARCH 2018, vol. 2018, 2018, pages 1205121, ISSN: 2314-6753**

**Description**

FIELD OF THE INVENTION

The instant invention relates to the field of closed-loop blood glucose control system for the controlled delivery of insulin to a patient. Such systems are also known as artificial pancreas.

BACKGROUND OF THE INVENTION

An artificial pancreas is a system that automatically regulates the insulin intake of a diabetic patient based on its blood glucose history, meal history, and insulin history.
In particular, the present invention relates to Model-based Predictive Control (MPC) systems, also known as predictive control systems, in which the determination of the dose of insulin to be injected is based on a prediction of the patient's future blood glucose level obtained by computing a physiological model describing the effect of insulin in the patient's body and its impact on the patient's glucose level.
The patent application US 2016/0354543 discloses methods and modules for using physiological (biometric) variables to advance the state of the artificial pancreas.
The article "Modeling the Effects of Exercise on Insulin Pharmacokinetics in 'Continuous Subcutaneous Insulin Infusion' Treated Type 1 Diabetes Patients" by Duun-Henriksen et. al. explores the effect of exercise on the insulin and plasma glucose dynamics in the development of an artificial pancreas.
The article "Model Predictive Control Algrorithms for Pen and Pump Insulin Administration" by Boiroux et. al. relates to different control strategies based on Model Predictive Control for an artificial pancreas.
It would be desirable to be able to improve the performance of model-based artificial pancreas, and more particularly to be able to improve the accuracy of the physiological model predictions in order to better estimate insulin requirements and reduce the risk of hyperglycemia or hypoglycemia.
The instant invention has notably for object to improve this situation. The PhD thesis of Dimitri Boiroux titled "Model Predictive Control Algorithms for Pen and Pump Insulin Administration" (20 October 2015 (2015-10-20), http://orbit.dtu.dk/files/57023470/ phd283_Boiroux_D.pdf) discloses different control strategies based on Model Predictive Control (MPC) for an artificial pancreas.

SUMMARY OF THE INVENTION

According to an aspect, the invention relates to an automated closed-loop blood glucose control system for the controlled delivery of insulin to a patient comprising:

    a continuous glucose-monitoring sensor configured to provide a plurality of glucose measurement values representative of a measured glucose level of the patient at an associated plurality of measurement times;
    a subcutaneous insulin delivery device configured to deliver exogenous insulin in a subcutaneous tissue of the patient in response to an insulin delivery control signal, in particular continuously infused insulin and/or bolus insulin; and
    a controller programmed to receive the glucose measurement values and provide the delivery control signal to the insulin delivery device,
    wherein the controller is able to determine the insulin delivery control signal on the basis of a predicted glucose level determined by computing a physiological model of glucose-insulin system in the patient comprising a system of differential equations describing the evolution of a plurality of state variables as a function of time, said variables containing at least an instantaneous on-board insulin level representative of a quantity of insulin present in the subcutaneous layer and an instantaneous plasma insulin level representative of a quantity of insulin in a plasma of the patient,
    wherein said physiological model includes a sub-model of subcutaneous insulin absorption kinetics modeling transport of insulin from the subcutaneous tissue to the plasma, said sub-model comprising at least one differential equation describing an absorption of insulin from the subcutaneous tissue to the plasma wherein an instantaneous absorption rate of insulin from the subcutaneous tissue to the plasma is a decreasing function of the instantaneous on-board insulin level.

This allows improving the accuracy of the physiological model predictions.
According to some aspects, one may also use one or more of the following features:

-    the controller is able to perform at least one prediction step for a tested insulin injection, said tested insulin injection comprising at least one value indicative of a quantity of insulin injected at a future time, said prediction step comprising

· computing a plurality of predicted glucose levels at a plurality of respective future time steps, by unrolling the physiological model over time using a set of pre-estimated model parameters and said tested insulin injection, and
· determining a cost associated to said plurality of predicted glucose levels;

- said cost is function of a time-restricted subset of the plurality of predicted glucose levels such that

· a first predicted glucose level of said time-restricted subset is associated to a first time step not closer than 30 minutes from a current time step and
· a last predicted glucose level of said time-restricted subset is associated to a last time step not further than 3 hours from a current time step;

- the controller determines at least one insulin delivery control signal by performing a cost optimization operation comprising a plurality of prediction steps for a plurality of respective tested insulin injection;
- the controller is able to perform a self-calibration operation comprising a determination of a set of pre-estimated model parameters on the basis of a plurality of glucose measurement values, a plurality of known insulin delivery control signals and at least one meal ingestion indicator;
- said set of pre-estimated model parameters comprises a current instantaneous absorption rate of insulin, a current instantaneous on-board insulin level and a current instantaneous plasma insulin level;
- the controller periodically performs said self-calibration operation and said cost optimization operation;
- said physiological model of glucose-insulin system relates the evolution of state variables in a plurality of compartments, in particular at least a sub-cutaneous compartment and a plasma/body compartment;
- the system further comprises a physiological sensor adapted to measure physiological data, and wherein the instantaneous absorption rate of insulin from the subcutaneous tissue to the plasma is a function of said physiological data,

Notably where the physiological sensor is a pulse monitoring sensor configured to provide a plurality of heart rate measurement values representative of a measured heart rate of the patient at an associated plurality of measurement times,
and wherein the instantaneous absorption rate of insulin from the subcutaneous tissue to the plasma is a increasing function of a heart rate of the patient.

According to another aspect, the invention relates to a method for the controlled delivery of insulin to a patient using an automated closed-loop blood glucose control system, the method comprising:

continuously monitoring glucose, using a sensor, to provide a plurality of glucose measurement values representative of a measured glucose level of the patient at an associated plurality of measurement times;
determining an insulin delivery control signal, using a controller, by computing a physiological model of glucose-insulin system in the patient comprising a system of differential equations describing the evolution of a plurality of state variables as a function of time, said variables containing at least an instantaneous on-board insulin level representative of a quantity of insulin present in the subcutaneous layer and an instantaneous plasma insulin level representative of a quantity of insulin in a plasma of the patient,
wherein said physiological model includes a sub-model of subcutaneous insulin absorption kinetics modeling transport of insulin from the subcutaneous tissue to the plasma, said sub-model comprising at least one differential equation describing an absorption of insulin from the subcutaneous tissue to the plasma wherein an instantaneous absorption rate of insulin from the subcutaneous tissue to the plasma is a decreasing function of the instantaneous on-board insulin level.

BRIEF DESCRIPTION OF THE DRAWINGS

Other characteristics and advantages of the invention will readily appear from the following description of several of its embodiments, provided as non-limitative examples, and of the accompanying drawings.
On the drawings:

Figure 1 schematically illustrates, in the form of a block diagram, an embodiment of an automated closed-loop blood glucose control system for the controlled delivery of insulin to a patient according to the invention;
Figure 2 is a simplified representation of a physiological model used in the system of figure 1 to predict blood glucose level of a patient;

Figure 3 is a diagram representing in greater detail an embodiment of the physiological model of figure 2;
Figure 4 is a diagram illustrating an example of a method for the controlled delivery of insulin to a patient using the automated closed-loop blood glucose control system of figure 1; and
Figure 5 is an explanatory diagram for an embodiment of the self-calibration operation.

On the different figures, the same reference signs designate like or similar elements.

DETAILED DESCRIPTION

For the sake of clarity, only elements which are useful for understanding the embodiments described are shown on the figures and detailed in the present description. In particular, the glucose-monitoring sensor and the insulin delivery device of the blood glucose control system are not specifically detailed since the embodiments of the present invention are compatible with all or a majority of the blood glucose measuring and insulin injection devices.

Physical embodiments of the controller of the described control system are also not described with excessive details, the realization of such a controller unit being within the scope of the skilled man given the functional explanations given in the present specification.

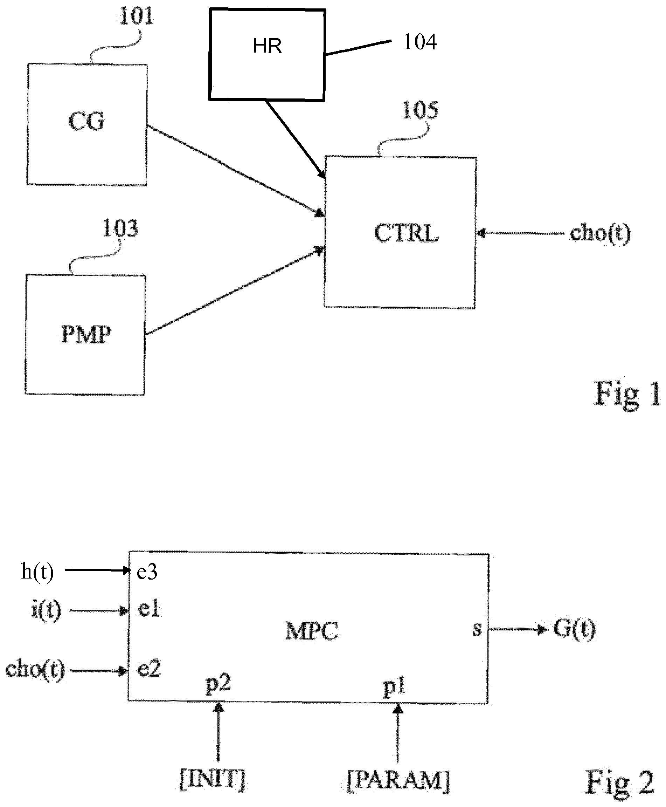

Figure 1 illustrates an example of an embodiment of an automated closed-loop blood glucose control system for the controlled delivery of insulin to a patient, in the form of a block diagram.

The system of figure 1 comprises a sensor 101 (CG) adapted to measure the blood glucose level of the patient. In normal operation, the sensor 101 can be positioned permanently on or in the body of the patient, for example at the level of its abdomen. The sensor 101 is for example a sensor of the "Continuous Glucose Monitoring" type (CGM), that is to say a sensor adapted to measure continuously (for example at least once per minute) the patient's blood glucose level. The sensor 101 is, for example, a subcutaneous blood glucose sensor.

The sensor may for instance comprise a disposable glucose sensor placed just under the skin, which may be worn for a few days and periodically replaced.

During the operation of the method and system of the present invention, the sensor 101 provides a plurality of glucose measurement values representative of a measured glucose level of the patient at an associated plurality of measurement times.

In the present description, "glucose level" is understood as a concentration of glucose in the blood, also called glycemia.

The system of figure 1 further comprises an insulin delivery device 103 (PMP), for example a subcutaneous injection device. The device 103 is for example an automatic injection device, like an insulin pump, comprising an insulin reservoir connected to an injection needle implanted under the skin of the patient. The pump is electrically commanded to inject controlled doses of insulin at determined times. In normal operation, the injection device 103 is positioned in or on the body of the patient, for example at the level of its abdomen.

During the operation of the method and system of the present invention, the insulin delivery device 103 delivers exogenous insulin in the subcutaneous tissue of the patient in response to an insulin delivery control signal. The exogenous insulin is in particular rapid-acting insulin. Rapid-acting insulin can be delivered by the insulin delivery device in two ways:

a bolus dose that is delivered to cover food eaten or to correct a high blood glucose level, or
a basal dose that is pumped continuously at an adjustable basal rate to deliver insulin needed between meals and at night.

In some embodiments, the system may also comprise a pulse monitoring sensor 104. The pulse monitoring sensor 104 is able to provide a plurality of heart rate measurement values h(t) representative of a heart rate of the patient measured at an associated plurality of measurement times. The pulse monitoring sensor 104 can be provided on an armband or waistband, for example. The sensor can be wirelessly connected to the remote controller 105 for the transfer of measured data thereto.

Alternatively, one may use another physiological sensor than a pulse monitoring sensor. Typical examples include a sensor measuring the electrical conductivity or the superficial temperature of the skin.

As illustrated on figure 1, the system further comprises a controller 105 (CTRL) which is a controller 105 connected to the glucose-monitoring sensor 101 and to the insulin delivery device 103 and, optionally, to the pulse monitoring sensor 104, for example by wired or radio (wireless) links.

During the operation of the method and system of the present invention, the controller 105 receives the blood glucose data of the patient measured by the sensor 101 and provides the delivery control signal to the insulin delivery device. The controller 105 may further receive, via a non-detailed user interface, indication of an amount of glucose ingested by the patient.

Such an indication on the amount of glucose is referenced as cho(t) and is in particular representative of the evolution of the ingestion of carbohydrate by the patient.

The controller 105 is adapted to determine the insulin delivery control signal to provide to the insulin delivery device.

To this aim, the controller 105 comprises a digital calculation circuit (not detailed), comprising, for example, a microprocessor. The controller 105 is, for example, a mobile device carried by the patient throughout the day and/or at night. One possible embodiment of the controller 105 may be a smartphone device configured to implement a method as described hereinafter.

The controller 105 is in particular adapted to determine a quantity of insulin to inject at at least one time step, taking into account a prediction of the future evolution of a blood glucose level of the patient as a function of time.

More precisely, the controller 105 determine a quantity of insulin to inject at at least one time step on the basis of a predicted glucose level determined by computing a physiological model of glucose-insulin system in the patient.

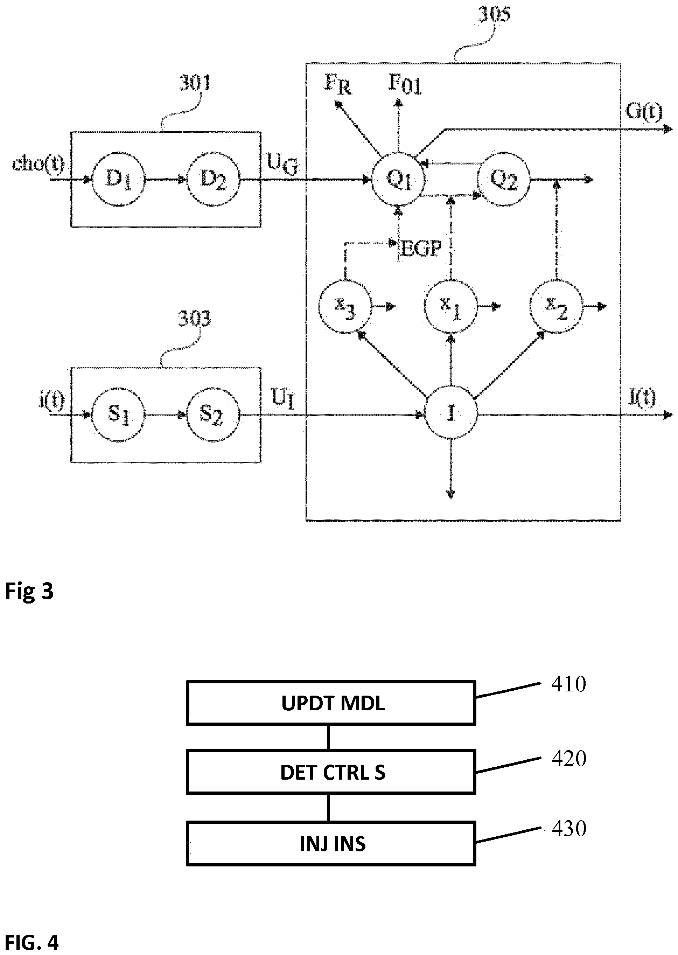

This determination operation is performed during an operation 420 illustrated on figure 4.

The controller 105 first determines a curve representative of an expected evolution of the patient's glucose level as a function of time over a future period during a step 421 illustrated on figure 4.

Taking this curve into account, the controller 105 determines the doses of insulin to be injected to the patient during the next period so that the glucose level of the patient remains within an acceptable range to limit the risk of hyperglycemia or hypoglycemia. As explained hereinafter, the glucose measurement values measured by the sensor 101 may for instance be used to perform a self-calibration operation of the physiological model.

Figure 2 is a schematic illustration of a physiological model of glucose-insulin system that may be implemented in the system of figure 1 to predict the evolution of glucose level of the patient.

The model is represented on figure 2 as a processing block comprising:

an input e1 on which is applied a signal i(t) indicative of an evolution, as a function of time t, of a quantity of exogenous insulin delivered in a subcutaneous layer of the patient by the insulin delivery device;
an input e2 on which is applied a signal cho(t) indicative of the evolution, as a function of time t, of the amount of glucose ingested by the patient, for example a quantity of carbohydrate ingested during a meal at a given time;
an input e3 on which is applied a signal h(t) indicative of the evolution, as a function of time t, of the heart rate of the patient; and
an output s providing a signal G(t) representative of the evolution, as a function of time t, of the patient's glucose level.

According to some embodiments, other physiological signals than the heart rate can be provided as input e3, as disclosed above.

The physiological model MPC is for example a compartmental model comprising, in addition to the input variables i(t) and cho(t) and the output variable G(t), a plurality of state variables corresponding to the instantaneous values of a plurality of physiological variables of the patient as they evolves over time.

The temporal evolution of the state variables is governed by a system of differential equations comprising a plurality of parameters represented in figure 2 by a vector [PARAM] applied to an input p1 of the MPC block.

The response of the physiological model may also be conditioned by the initial values assigned to the state variables, which is represented on figure 2 by a vector [INIT] applied to an input p2 of the MPC block.

Figure 3 is a diagram which represents in greater detail a non-limiting example of a physiological model implemented in an embodiment of the system of figure 1.

This exemplary model is known as the Hovorka model and is described for instance in "Nonlinear model predictive control of glucose concentration in subjects with type 1 diabetes" by Roman Hovorka et al. (Physiol Meas., 2004; 25: 905-920) and in "Partitioning glucose distribution/transport, disposal, and endogenous production during IVGTT" by Roman Hovorka et al. (Physical Endocrinol Metab 282: E992-E1007, 2002).

The physiological model illustrated on figure 3 comprises a first bi-compartmental sub-model 301 describing the effect of glucose intake on the rate of onset of glucose in blood plasma.

Sub-model 301 takes as input a quantity of ingested glucose cho(t), for example in mmol/min, and provides as an output a rate $U_G$ of absorption of glucose in the blood plasma, for example in mmol/min.

In this model, sub-model 301 comprises two state variables $D_1$ and $D_2$ that respectively corresponds to glucose masses, for example in mmol, respectively in the first and the second compartment.

The model of figure 3 also comprises a second bi-compartmental sub-model 303 describing the absorption of exogenous insulin delivered to the patient in the blood plasma. Sub-model 303 takes a quantity of exogenous insulin i(t) delivered in the subcutaneous tissue of the patient as an input, for example in mU/min, and provides as an output a rate $U_I$ of absorption of insulin in the plasma, in MU/min.

The sub-model 303 may for instance comprise two state variables $S_1$ and $S_2$, respectively corresponding to on-board insulin which are insulin masses respectively in the first and the second compartments modeling a subcutaneous compartment representative of the sub-cutaneous tissue of the patient. The instantaneous on-board insulin level of the state variables $S_1$ and $S_2$ may for example be expressed in mmol.

The model of figure 3 may further comprise a third sub-model 305 describing the regulation of glucose by the patient's body. This sub-model 305 takes as inputs the absorption rates $U_G$, $U_I$ of glucose and insulin, and gives as output the blood glucose level G(t), i.e. the concentration of glucose in the plasma, for example in mmol/l.

The sub-model 305 is thus a model of a plasma/body compartment of the patient, i.e. a model of the kinetic and chemical evolution of glucose and insulin in the plasma and the body of the patient.

By "plasma and body of the patient", it is meant the body of the patient with the exception of the sub-cutaneous tissues.

In this example, the submodel 305 comprises six state variables Q1, Q2, x3, x1, x2, I.

Variables Q1 and Q2 respectively correspond to masses of glucose respectively in the first and the second compartments, for example mmol.

Variables x1, x2, x3 are dimensionless variables respectively representing each of three respective actions of insulin on the kinetics of glucose.

Finally, variable I is an instantaneous plasma insulin level, i.e. corresponds to insulinemia which is a concentration of insulin in the blood plasma. The instantaneous plasma insulin level is for example expressed in mU/l.

The Hovorka model is governed by the following system of equations:

$$\frac{dS_1}{dt} = i(t) - k_a . S_1(t)$$

$$\frac{dS_2}{dt} = k_a . S_1(t) - k_a . S_2(t)$$

$$\frac{dI}{dt} = \frac{k_a . S_2(t)}{V_I} - k_e . I(t)$$

$$\frac{dD_1}{dt} = cho(t) - \frac{D_1(t)}{t_{max}}$$

$$\frac{dD_2}{dt} = \frac{D_1(t)}{t_{max}} - \frac{D_2(t)}{t_{max}}$$

$$U_G = \frac{D_2(t)}{t_{max}}$$

$$\frac{dQ_1}{dt} = -\left[\frac{F_{01}^c}{V_G . G(t)} + x_1(t)\right] . Q_1(t) + k_{12} Q_2(t) - F_R + EGP_0 . [1 - x_3(t)] + U_G(t)$$

$$\frac{dQ_2}{dt} = x_1(t) . Q_1(t) - [k_{12} + x_2(t)] . Q_2(t)$$

$$\frac{dx_1}{dt} = -k_{a1} . x_1(t) + k_{b1} . I(t)$$

$$\frac{dx_2}{dt} = -k_{a2} . x_2(t) + k_{b2} . I(t)$$

$$\frac{dx_3}{dt} = -k_{a3}.x_3(t) + k_{b3}.I(t)$$

$$G(t) = \frac{Q_1(t)}{V_G}$$

with

$$F_{01}^c = \frac{F_{01}.G(t)}{0.85 \cdot (G(t) + 1..0)}$$

and

$$F_R = \begin{cases} 0.003(G - 9).V_G & \text{if } G > 9 \\ 0 & \text{otherwise} \end{cases}$$

This system of differential equations comprises fifteen parameters $V_G$, $F_{01}$, $k_{12}$, $F_R$, $EGP_0$, $k_{b1}$, $k_{a1}$, $k_{b2}$, $k_{a2}$, $k_{b3}$, $k_{a3}$, $k_a$, $V_I$, $k_e$ and $t_{max}$ with the following meaning:

$V_G$ corresponds to a volume of distribution of the glucose, for example in liters,
$F_{01}$ corresponds to a non-insulin-dependent glucose transfer rate, for example in mmol/min,
$k_{12}$ corresponds to a transfer rate between the two compartments of sub Model 305, for example in min$^{-1}$,
$k_{ai}$, $k_{a2}$, $k_{a3}$ correspond to an insulin deactivation rate constants, for example in min$^{-1}$,
$F_R$ corresponds to a urinary excretion of glucose, for example in mmol/min,
$EGP_0$ corresponds to an endogenous production of glucose, for example in min$^{-1}$,
$k_{b1}$, $k_{b2}$ and $k_{b3}$ correspond to insulin activation insulin injected subcutaneously, for example in min-1,
$V_I$ corresponds to the volume of distribution of the insulin, for example in liters,
$k_e$ corresponds to a plasma elimination rate of insulin, for example in min$^{-1}$, and
$t_{max}$ corresponds to a time to peak glucose absorption ingested by the patient, for example in min.

These fifteen parameters correspond to the vector [PARAM] illustrated on figure 2.
This model further comprises ten state variables $D_1$, $D_2$, $S_1$, $S_2$, $Q_1$, $Q_2$, $X_1$, $X_2$, $X_3$ and I, which are initiated to a vector [INIT] of initial values corresponding to values of these variables at a time step t0 corresponding a beginning of the simulation of the patient's behavior by the model.
The system and method of the invention may also uses more simple physiological models than the Hovorka model described above, in particular a compartmental model with a smaller number of state variables and a reduced number of parameters compared to the Hovorka model.
In some embodiment of the invention, several physiological models may be stored in the controller and the predictions of said models may be compared together or may be used independently, for example depending on a state of the controller or a confidence indicator that may be computed for the predictions of said models.
In particular, the embodiments described are not limited to the Hovorka physiological model but are compatible with any physiological model describing the assimilation of insulin by a patient's body and its effect on the patient's blood glucose level.
One example of another physiological model is the Cobelli model described in "A System Model of Oral Glucose Absorption: Validation on Gold Standard Data" by Chiara Dalla Man et al. in IEEE TRANSACTIONS ON BIOMEDICAL ENGINEERING, VOL 53, NO 12, DECEMBER 2006.
Such models are known from the literature.
In these models, the absorption rate of insulin from the subcutaneous tissue to the plasma is constant.
There is such a need to improve the accuracy of predicting the absorption of insulin from the subcutaneous tissue to the plasma.
To this aim, the absorption rate of insulin from the subcutaneous tissue to the plasma $k_a(t)$ is a function of the instantaneous on-board insulin level $S_2(t)$.
In particular, the instantaneous absorption rate $k_a(t)$ of insulin from the subcutaneous tissue to the plasma may be a decreasing function of the instantaneous on-board insulin level $S_2(t)$.

In one embodiment of the invention, the instantaneous absorption rate $k_a(t)$ of insulin from the subcutaneous tissue may be proportional to an inverse of the instantaneous on-board insulin level $S_2(t)$.

This way, it is possible to take into account the absorption kinetics of the transport of insulin from the subcutaneous tissue to the plasma and in particular the subcutaneous blood flow.

In some embodiment of the invention, the instantaneous absorption rate of insulin $k_a(t)$ may be a function of a heart rate of the patient.

In particular, the instantaneous absorption rate of insulin $k_a(t)$ may be an increasing function of a heart rate of the patient.

According to some embodiments, the instantaneous absorption rate of insulin $k_a(t)$ may be a function of other physiological signals than the heart rate, as disclosed above.

Using the above described model, the controller 105 perform can determine an insulin delivery control signal by performing a cost optimization operation as illustrated on figure 4.

The cost optimization operation advantageously comprises a plurality of prediction steps for a plurality of respective tested insulin injection.

A tested insulin injection is a set comprising at least one value indicative of a quantity of insulin injected at a future time. The tested insulin injection may comprise a plurality of M values indicative of a quantity of insulin injected at a respective future time of a plurality of M future time step, with M being an integer strictly greater than 1.

A prediction step for a tested insulin injection comprises the sub-steps of:

computing a plurality of predicted glucose levels at a plurality of respective future time steps by unrolling the physiological model over time, and
determining a cost associated to said plurality of predicted glucose levels.

The unrolling of the physiological model over time is performed using the tested insulin injection and a set of pre-estimated model parameters that can be in particular determined during a self-calibration operation as detailed further below.

The cost associated to the predicted glucose levels is for instance related to a distance of each predicted glucose level with a target glucose level.

The target glucose level may be pre-defined and personalized for the patient. The target glucose level may be comprised between 100 and 130 mg/dL.

The cost function can be for example a quadratic function of a difference between a predicted glucose level and a target glucose level.

Advantageously, the cost function may be asymmetrical and in particular may penalize more strongly glucose level lower than the target glucose level than glucose level higher than the target glucose level.

In some embodiment of the invention, the cost associated to the predicted glucose levels is a function of a time-restricted subset of the plurality of predicted glucose levels.

Such a time-restricted subset may be such that the predicted glucose levels are only considered in a restricted range, in which the glucose level of the patient may be considered controllable by the system and method of the invention.

For example, glucose level in a short future period close to the current time may not be considered controllable since the kinetic of sub-cutaneous delivered insulin is too slow to have an effect in the near future. On the other hand, glucose level in a far future period, far-away from the current time, may also not be considered controllable since the uncertainty on the parameters, states-variables and meal uptake is too high to have a reliable prediction of the glucose level.

Thus, a first predicted glucose level of said time-restricted subset may be associated to a first time step not closer than 30 minutes from a current time step and a last predicted glucose level of said time-restricted subset may be associated to a last time step not further than 3 hours from a current time step.

The controller can thus determine a quantity of insulin to inject by performing a cost optimization operation comprising a plurality of prediction steps for a plurality of respective tested insulin injection.

The controller 105 may in particular select a quantity of insulin to inject minimizing the cost associated to the predicted glucose levels.

The controller 105 can then generate an insulin delivery control signal from the quantity of insulin to inject and send the delivery control signal to the insulin delivery device 103.

The delivery device can then inject insulin 430 on the basis of the delivery control signal.

Among the parameters of the [PARAM] vector, some parameters may be considered as constant for a given patient, for example parameters $k_{12}$, $k_{a1}$, $k_{a2}$, $k_{a3}$, $k_e$, $V_I$, $V_G$ and $t_{max}$. Other parameters, referred to hereinafter as time-dependent parameters, may change over time, for example the parameters $k_a$, $k_{b1}$, $k_{b2}$, $k_{b3}$, $EGP_0$, $F_{01}$ and $F_R$.

Because of this variation of some parameters, it is in practice necessary to regularly recalibrate or self-calibration the model in use, for example every 1 to 20 minutes, to ensure that the predictions remain accurate.

The self-calibration of the model should advantageously be carried out automatically by the system, in particular without physically measuring the parameters of the model.

Figure 4 is a diagram illustrating an example of a method according to the invention.

This method comprises an operation 410 of self-calibration of the model, which may for example be repeated at regular intervals, for example every 1 to 20 minutes.

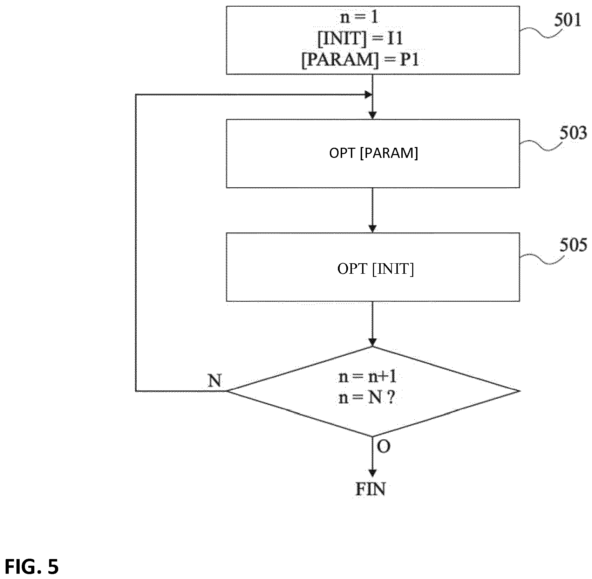

This self-calibration operation is illustrated in greater detail on figure 5.

During this self-calibration operation, the controller 105 implements a determination of a set of pre-estimated model parameters taking into account the glucose measurement values, known insulin delivery control signals and at least one meal ingestion indicator during a past period, for example a period of 1 to 10 hours preceding the self-calibration operation.

For example, during the self-calibration operation, the controller 105 may simulates the behavior of the patient over the past period using the physiological model and taking into account glucose ingestions and exogenous insulin injections during this period. The controller 105 may then compares the glucose level curve estimated using the model to the actual glucose measurements by the sensor over the same period.

The controller 105 may then determine a set of predetermined values for a set of model parameters that leads to minimizing a cost function over the observation period.

In a similar manner, the self-calibration operation may comprise an estimation of the initial states vector [INIT] of the state variables of the model, as it will now be described in relation with figure 5 which is illustrating an example of an embodiment of a method according to the invention.

The self-calibration operation comprises a step 501 during which a set of parameters of the model is initialized to a first set of values P1 of the parameter vector [PARAM].

The set P1 corresponds, for example, to the values taken by the parameters [PARAM] during a previous self-calibration operation. In a variant, the set of values P1 may be a predefined reference set corresponding, for example, to mean values of the parameters [PARAM] over a reference period.

During a step 501, a set of state variables values may also be initialized to a first set of values I1 forming an initial state vector [INIT].

The set of values I1 may be determined analytically, for example by assuming a stationary state of the patient in a period preceding the calibration phase and coinciding an estimated blood glucose level at time t0 with an actual glucose level measurement at that time.

In a subsequent step 503, the controller 105 may fix the set of initial states [INIT] to its current state and search for a set of values for the parameters of the model to minimizing a cost, for example an error between an estimated glucose level curve predicted by the model and an actual glucose level curve during a past observation period.

An example of such a cost function may be written as:

$$m = \frac{1}{\Delta T} \sum_{t=t_0}^{t_0 + \Delta T} |g(t) - \hat{g}(t)|^2$$

where t is a discretized time, $t_0$ correspond to an initial time of the past observation period, $t_0 + \Delta T$ correspond to an end of said past observation period (for instance the beginning of the self-calibration operation), $g(t)$ is a curve determined from the glucose level measurements and $\tilde{g}(t)$ is a curve determined from the glucose level predicted by the model.

At the end of this step, the [PARAM] vector is updated with the new estimated values.

In a step 505 subsequent to step 503, the controller 105 then searches for a set of initial state values, setting the parameter set [PARAM] to its current state, here again by minimizing a cost such as the above described error between the estimated glucose level curve predicted by the model and an actual glucose level curve determined during a past observation period.

At the end of this step, the vector [INIT] is updated with the new estimated values.

In some embodiments, steps 503 and 505 may be reiterated a number N of times, where N is an integer greater than 1 that may be predetermined.

The values of the parameters and the initial states of the model are then updated to the corresponding values of the vectors [PARAM] and [INIT] at the end of the Nth iteration of steps 503 and 505.

In a variant, the number of iterations of steps 503 and 505 may not be predetermined and may be adjusted by taking into account the evolution of cost function over the successive iterations.

The algorithms that can be used to find the optimal values in steps 503 and 505 are not described in details in the present application. The method described in the present specification is compatible with the algorithms commonly used in various domains to solve optimization problems by minimizing a cost function.

**Claims**

1. An automated closed-loop blood glucose control system for the controlled delivery of insulin to a patient comprising:

   a continuous glucose-monitoring sensor (101) configured to provide a plurality of glucose measurement values representative of a measured glucose level of the patient at an associated plurality of measurement times;
   a subcutaneous insulin delivery device (103) configured to deliver exogenous insulin in a subcutaneous tissue of the patient in response to an insulin delivery control signal, in particular continuously infused insulin and/or bolus insulin; and
   a controller (105) programmed to receive the glucose measurement values and provide the delivery control signal to the insulin delivery device,
   wherein the controller (105) is able to determine the insulin delivery control signal on the basis of a predicted glucose level determined by computing a physiological model of glucose-insulin system in the patient, said model describing the effect of insulin in the patient's body and its impact on the patient's glucose level and comprising a system of differential equations describing the evolution of a plurality of state variables (D1, D2, S1, S2, Q1, Q2, x1, x2, x3, I) as a function of time, said variables containing at least an instantaneous on-board insulin level representative of a quantity of insulin present in the subcutaneous layer and an instantaneous plasma insulin level representative of a quantity of insulin in a plasma of the patient,
   wherein said physiological model includes a sub-model of subcutaneous insulin absorption kinetics modeling transport of insulin from the subcutaneous tissue to the plasma, said sub-model comprising at least one differential equation describing an absorption of insulin from the subcutaneous tissue to the plasma **characterized in that** the instantaneous absorption rate of insulin from the subcutaneous tissue to the plasma is a decreasing function of the instantaneous on-board insulin level.

2. The system according to anyone of claim 1, wherein the controller (105) is able to perform at least one prediction step for a tested insulin injection, said tested insulin injection comprising at least one value indicative of a quantity of insulin injected at a future time, said prediction step comprising

   computing a plurality of predicted glucose levels at a plurality of respective future time steps, by unrolling the physiological model over time using a set of pre-estimated model parameters and said tested insulin injection, and determining a cost associated to said plurality of predicted glucose levels.

3. The system according to claim 2, wherein said cost is function of a time-restricted subset of the plurality of predicted glucose levels such that

   a first predicted glucose level of said time-restricted subset is associated to a first time step not closer than 30 minutes from a current time step and
   a last predicted glucose level of said time-restricted subset is associated to a last time step not further than 3 hours from a current time step.

4. The system according to anyone of claim 2 or 3, wherein the controller determines at least one insulin delivery control signal by performing a cost optimization operation comprising a plurality of prediction steps for a plurality of respective tested insulin injection.

5. The system according to anyone of claim 1 to 4, wherein the controller (105) is able to perform a self-calibration operation comprising a determination of a set of pre-estimated model parameters on the basis of a plurality of glucose measurement values, a plurality of known insulin delivery control signals and at least one meal ingestion indicator.

6. The system according to claim 5, wherein said set of pre-estimated model parameters comprises a current instantaneous absorption rate of insulin, a current instantaneous on-board insulin level and a current instantaneous plasma insulin level.

7. The system according to anyone of claim 2 to 6, wherein the controller periodically performs said self-calibration operation and said cost optimization operation.

8. The system according to anyone of claim 1 to 7, wherein said physiological model of glucose-insulin system relates the evolution of state variables in a plurality of compartments, in particular at least a sub-cutaneous compartment and a plasma/body compartment.

9. A method for determining the controlled delivery of insulin to a patient using an automated closed-loop blood glucose control system, the method comprising:

continuously monitoring glucose, using a sensor, to provide a plurality of glucose measurement values representative of a measured glucose level of the patient at an associated plurality of measurement times;
receiving, by a controller, the glucose measurement values,
determining, by the controller, an insulin delivery control signal by computing a physiological model of glucose-insulin system in the patient, said model describing the effect of insulin in the patient's body and its impact on the patient's glucose level and comprising a system of differential equations describing the evolution of a plurality of state variables (D1, D2, S1, S2, Q1, Q2, x1, x2, x3, I) as a function of time, said variables containing at least an instantaneous on-board insulin level representative of a quantity of insulin present in the subcutaneous layer and an instantaneous plasma insulin level representative of a quantity of insulin in a plasma of the patient,
wherein said physiological model includes a sub-model of subcutaneous insulin absorption kinetics modeling transport of insulin from the subcutaneous tissue to the plasma, said sub-model comprising at least one differential equation describing an absorption of insulin from the subcutaneous tissue to the plasma **characterized in that** the instantaneous absorption rate of insulin from the subcutaneous tissue to the plasma is a decreasing function of the instantaneous on-board insulin level.

**Patentansprüche**

1. ein automatisiertes Blutzuckerkontrollsystem mit geschlossenem Regelkreis für die kontrollierte Abgabe von Insulin an einen Patienten, umfassend:

einen kontinuierlichen Glukoseüberwachungssensor (101), der so konfiguriert ist, dass er eine Vielzahl von Glukosemesswerten liefert, die für einen gemessenen Glukosespiegel des Patienten bei einer zugehörigen Anzahl von Messzeiten repräsentativ sind;
eine Vorrichtung zur subkutanen Insulinabgabe (103), die so konfiguriert ist, dass sie exogenes Insulin in ein subkutanes Gewebe des Patienten als Reaktion auf ein Kontrollsignal für die Insulinabgabe, insbesondere kontinuierlich infundiertes Insulin und/oder Bolusinsulin, abgibt; und
eine Steuerung (105), die so programmiert ist, dass sie die Glukosemesswerte empfängt und das Abgabesteuersignal an die Insulinabgabevorrichtung liefert,
wobei die Steuerung (105) in der Lage ist, das Steuersignal für die Insulinabgabe auf der Grundlage eines vorhergesagten Glukosespiegels zu bestimmen, der durch Berechnung eines physiologischen Modells des Glukose-Insulin-Systems bei dem Patienten bestimmt wird, wobei das Modell die Wirkung von Insulin im Körper des Patienten und seine Auswirkungen auf den Glukosespiegel des Patienten beschreibt und ein System von Differentialgleichungen umfasst, das die Entwicklung einer Vielzahl von Zustandsvariablen (D1, D2, S1, S2, Q1, Q2, x1, x2, x3, I) als Funktion der Zeit, wobei die Variablen mindestens einen momentanen On-Board-Insulinspiegel enthalten, der für eine in der subkutanen Schicht vorhandene Insulinmenge repräsentativ ist, und einen momentanen Plasmainsulinspiegel, der für eine Insulinmenge in einem Plasma des Patienten repräsentativ ist,
wobei das physiologische Modell ein Untermodell der subkutanen Insulinabsorptionskinetik umfasst, das den Transport von Insulin aus dem Unterhautgewebe zum Plasma modelliert, wobei das Untermodell mindestens eine Differentialgleichung umfasst, die eine Absorption von Insulin aus dem Unterhautgewebe in das Plasma beschreibt, **dadurch gekennzeichnet, dass** die momentane Absorptionsrate von Insulin aus dem Unterhautgewebe zum Plasma eine abnehmende Funktion des momentanen On-Board-Insulinspiegels ist.

2. System nach Anspruch 1, wobei der Controller (105) in der Lage ist, mindestens einen Vorhersageschritt für eine getestete Insulininjektion durchzuführen, wobei die getestete Insulininjektion mindestens einen Wert umfasst, der auf eine zu einem späteren Zeitpunkt injizierte Insulinmenge hinweist, wobei der Vorhersageschritt umfasst

Berechnung einer Vielzahl von vorhergesagten Glukosespiegeln in einer Vielzahl von jeweiligen zukünftigen Zeitschritten, indem das physiologische Modell über die Zeit unter Verwendung eines Satzes von vorgeschätzten Modellparametern und der getesteten Insulininjektion abgerollt wird, und
Bestimmung der Kosten, die mit der Vielzahl der vorhergesagten Glukosespiegel verbunden sind.

3. System nach Anspruch 2, wobei die Kosten eine Funktion einer zeitlich begrenzten Teilmenge der Vielzahl der vorhergesagten Glukosespiegel sind, so dass

ein erster vorhergesagter Glukosespiegel dieser zeitlich begrenzten Teilmenge mit einem ersten Zeitschritt verbunden ist, der nicht näher als 30 Minuten von einem aktuellen Zeitschritt entfernt ist, und
Ein letzter vorhergesagter Glukosespiegel dieser zeitlich begrenzten Teilmenge ist mit einem letzten Zeitschritt verbunden, der nicht weiter als 3 Stunden von einem aktuellen Zeitschritt entfernt ist.

4. System nach einem der Ansprüche 2 oder 3, wobei das Steuergerät mindestens ein Insulinabgabesteuersignal bestimmt, indem es eine Kostenoptimierungsoperation durchführt, die eine Vielzahl von Vorhersageschritten für eine Vielzahl von jeweils getesteten Insulininjektionen umfasst.

5. System nach Anspruch 1 bis 4, wobei das Steuergerät (105) in der Lage ist, eine Selbstkalibrierung durchzuführen, die eine Bestimmung eines Satzes von vorgeschätzten Modellparametern auf der Grundlage einer Vielzahl von Glukosemesswerten, einer Vielzahl bekannter Insulinabgabesteuersignale und mindestens eines Indikators für die Nahrungsaufnahme umfasst.

6. System nach Anspruch 5, wobei der Satz von vorgeschätzten Modellparametern die aktuelle momentane Absorptionsrate von Insulin, den aktuellen momentanen On-Board-Insulinspiegel und den aktuellen momentanen Plasmainsulinspiegel umfasst.

7. System nach Anspruch 2 bis 6, wobei das Steuergerät periodisch den Selbstkalibrierungsvorgang und den Kostenoptimierungsvorgang durchführt.

8. System nach Anspruch 1 bis 7, wobei das physiologische Modell des Glukose-Insulin-Systems die Entwicklung von Zustandsvariablen in einer Vielzahl von Kompartimenten, insbesondere zumindest einem subkutanen Kompartiment und einem Plasma-/Körperkompartiment, in Beziehung setzt.

9. Verfahren zur Bestimmung der kontrollierten Abgabe von Insulin an einen Patienten unter Verwendung eines automatisierten Blutzuckerkontrollsystems mit geschlossenem Regelkreis, wobei das Verfahren Folgendes umfasst:

kontinuierliche Überwachung der Glukose unter Verwendung eines Sensors, um eine Vielzahl von Glukosemesswerten bereitzustellen, die für einen gemessenen Glukosespiegel des Patienten zu einer entsprechenden Anzahl von Messzeiten repräsentativ sind;
Empfangen der Glukosemesswerte durch ein Steuergerät,
Bestimmung eines Insulinabgabe-Steuersignals durch den Controller durch Berechnung eines physiologischen Modells des Glukose-Insulin-Systems im Patienten, wobei dieses Modell die Wirkung von Insulin im Körper des Patienten und seine Auswirkungen auf den Glukosespiegel des Patienten beschreibt und ein System von Differentialgleichungen umfasst, das die Entwicklung einer Vielzahl von Zustandsvariablen beschreibt (D1, D2, S1, S2, Q1, Q2, x1, x2, x3, I) als Funktion der Zeit, wobei die Variablen mindestens einen momentanen On-Board-Insulinspiegel enthalten, der für eine in der subkutanen Schicht vorhandene Insulinmenge repräsentativ ist, und einen momentanen Plasmainsulinspiegel, der für eine Insulinmenge in einem Plasma des Patienten repräsentativ ist,
wobei das physiologische Modell ein Untermodell der subkutanen Insulinabsorptionskinetik umfasst, das den Transport von Insulin vom Unterhautgewebe zum Plasma modelliert, wobei das Untermodell mindestens eine Differentialgleichung umfasst, die eine Absorption von Insulin aus dem Unterhautgewebe in das Plasma beschreibt, **dadurch gekennzeichnet, dass** die momentane Absorptionsrate von Insulin aus dem Unterhautgewebe zum Plasma eine Funktion des momentanen On-Board-Insulinspiegels ist.

**Revendications**

1. Système automatisé de contrôle de la glycémie en boucle fermée pour l'administration contrôlée d'insuline à un patient comprenant :

un capteur de surveillance du glucose en continu (101) configuré pour fournir une pluralité de valeurs de mesure du glucose représentatives d'un taux de glucose mesuré du patient à une pluralité associée de temps de mesure ;
dispositif d'administration sous-cutanée d'insuline (103) configuré pour délivrer de l'insuline exogène dans un tissu sous-cutané du patient en réponse à un signal de contrôle de l'administration d'insuline, en particulier de l'insuline perfusée en continu et/ou de l'insuline en bolus ; et

un contrôleur (105) programmé pour recevoir les valeurs de mesure du glucose et fournir le signal de commande d'administration au dispositif d'administration d'insuline,

dans lequel le contrôleur (105) est capable de déterminer le signal de commande de l'administration d'insuline sur la base d'un taux de glucose prédit déterminé par le calcul d'un modèle physiologique du système glucose-insuline chez le patient, ledit modèle décrivant l'effet de l'insuline dans le corps du patient et son impact sur le taux de glucose du patient et comprenant un système d'équations différentielles décrivant l'évolution d'une pluralité de variables d'état (D1, D2, S1, S2, Q1, Q2, x1, x2, x3, I) en fonction du temps, lesdites variables contenant au moins un taux d'insuline embarqué instantané représentatif d'une quantité d'insuline présente dans la couche sous-cutanée et un taux d'insuline plasmatique instantané représentatif d'une quantité d'insuline dans un plasma du patient,

dans lequel ledit modèle physiologique comprend un sous-modèle de cinétique d'absorption sous-cutanée de l'insuline modélisant le transport de l'insuline du tissu sous-cutané vers le plasma, ledit sous-modèle comprenant au moins une équation différentielle décrivant une absorption de l'insuline du tissu sous-cutané vers le plasma, **caractérisé en ce que** le taux d'absorption instantanée de l'insuline du tissu sous-cutané vers le plasma est une fonction décroissante du taux d'insuline instantané embarqué.

2. Système selon l'une quelconque des revendications 1, dans lequel le contrôleur (105) est capable d'effectuer au moins une étape de prédiction pour une injection d'insuline testée, ladite injection d'insuline testée comprenant au moins une valeur indicative d'une quantité d'insuline injectée à un moment ultérieur, ladite étape de prédiction comprenant

calculer une pluralité de taux de glucose prédits à une pluralité d'intervalles de temps futurs respectifs, en déroulant le modèle physiologique au fil du temps à l'aide d'un ensemble de paramètres de modèle pré-estimés et de ladite injection d'insuline testée, et

déterminer un coût associé à ladite pluralité de taux de glucose prédits.

3. Système selon la revendication 2, dans lequel ledit coût est fonction d'un sous-ensemble limité dans le temps de la pluralité des taux de glucose prédits tels que

un premier taux de glucose prévu dudit sous-ensemble limité dans le temps est associé à un premier pas de temps non situé à moins de 30 minutes d'un pas de temps courant et

Un dernier taux de glucose prévu dudit sous-ensemble limité dans le temps est associé à un dernier pas de temps non situé à plus de 3 heures à partir d'un pas de temps actuel.

4. Système selon l'une quelconque des revendications 2 ou 3, dans lequel le contrôleur détermine au moins un signal de commande d'administration d'insuline en effectuant une opération d'optimisation des coûts comprenant une pluralité d'étapes de prédiction pour une pluralité d'injections d'insuline testées respectives.

5. Système selon l'une quelconque des revendications 1 à 4, dans lequel le contrôleur (105) est capable d'effectuer une opération d'auto-étalonnage comprenant la détermination d'un ensemble de paramètres de modèle pré-estimés sur la base d'une pluralité de valeurs de mesure de glucose, d'une pluralité de signaux de commande d'administration d'insuline connus et d'au moins un indicateur d'ingestion de repas.

6. Système selon la revendication 5, dans lequel ledit ensemble de paramètres de modèle pré-estimés comprend un taux d'absorption instantané actuel de l'insuline, un taux d'insuline embarqué instantané actuel et un taux d'insuline plasmatique instantané actuel.

7. Système selon l'une quelconque des revendications 2 à 6, dans lequel le contrôleur effectue périodiquement ladite opération d'auto-étalonnage et ladite opération d'optimisation des coûts.

8. Système selon l'une quelconque des revendications 1 à 7, dans lequel ledit modèle physiologique du système glucose-insuline met en relation l'évolution des variables d'état dans une pluralité de compartiments, en particulier au moins un compartiment sous-cutané et un compartiment plasma/corps.

9. Procédé de détermination de l'administration contrôlée d'insuline à un patient à l'aide d'un système automatisé de contrôle de la glycémie en boucle fermée, le procédé comprenant :

surveillance continue du glucose, à l'aide d'un capteur, pour fournir une pluralité de valeurs de mesure du glucose représentatives d'un taux de glucose mesuré du patient à une pluralité associée de temps de mesure ; la réception, par un contrôleur, des valeurs de mesure du glucose,

déterminant, par le contrôleur, un signal de contrôle de l'administration d'insuline par calcul d'un modèle physiologique du système glucose-insuline chez le patient, ledit modèle décrivant l'effet de l'insuline dans l'organisme du patient et son impact sur le taux de glucose du patient et comprenant un système d'équations différentielles décrivant l'évolution d'une pluralité de variables d'état (D1, D2, S1, S2, Q1, Q2, x1, x2, x3, I) en fonction du temps, lesdites variables contenant au moins un taux d'insuline embarqué instantané représentatif d'une quantité d'insuline présente dans la couche sous-cutanée et un taux d'insuline plasmatique instantané représentatif d'une quantité d'insuline dans un plasma du patient,

dans lequel ledit modèle physiologique comprend un sous-modèle de cinétique d'absorption sous-cutanée de l'insuline modélisant le transport de l'insuline du tissu sous-cutané vers le plasma, ledit sous-modèle comprenant au moins une équation différentielle décrivant une absorption de l'insuline du tissu sous-cutané vers le plasma, **caractérisé en ce que** le taux d'absorption instantanée de l'insuline du tissu sous-cutané vers le plasma est fonction du taux d'insuline instantané embarqué.

Fig 1

Fig 2

**Fig 3**

**FIG. 4**

**FIG. 5**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20160354543 A

### Non-patent literature cited in the description

- **DUUN-HENRIKSEN.** Modeling the Effects of Exercise on Insulin Pharmacokinetics. *Continuous Subcutaneous Insulin Infusion' Treated Type 1 Diabetes Patients*
- **BOIROUX.** *Model Predictive Control Algrorithms for Pen and Pump Insulin Administration*
- *Model Predictive Control Algorithms for Pen and Pump Insulin Administration,* 20 October 2015, http://orbit.dtu.dk/files/57023470/ phd283_Boiroux_D.pdf
- **ROMAN HOVORKA et al.** Nonlinear model predictive control of glucose concentration in subjects with type 1 diabetes. *Physiol Meas.,* 2004, vol. 25, 905-920
- **ROMAN HOVORKA et al.** Partitioning glucose distribution/transport, disposal, and endogenous production during IVGTT. *Physical Endocrinol Metab,* 2002, vol. 282, E992-E1007
- **CHIARA DALLA MAN et al.** A System Model of Oral Glucose Absorption: Validation on Gold Standard Data. *IEEE TRANSACTIONS ON BIOMEDICAL ENGINEERING,* December 2006, vol. 53 (12